# EUROPEAN PATENT SPECIFICATION

(11) **EP 2 377 482 B1**
(45) Date of publication and mention of the grant of the patent: **18.12.2013**
(21) Application number: 11172575.0
(22) Date of filing: 01.10.2007
(51) Int. Cl.: A61B 18/20, A61N 5/067, H01S 3/094, H01S 3/16

(54) **A laser system for treatment of skin**
Lasersystem zur Behandlung der Haut
Système à laser pour traitement de la peau

(30) Priority: 29.09.2006 US 848083 P
(43) Date of publication of application: 19.10.2011
(62) Divisional of application: 07843620.1
(73) Proprietor: CANDELA CORPORATION, Wayland, MA 01778 (US)
(72) Inventor: Jones, Christopher J., Leicester, MA Massachusetts 01524 (US); Bhawalkar, Jayant D., Brighton, MA Massachusetts 02135 (US)
(74) Representative: Lawrence, John

(56) References cited:
- US-A- 5 757 839

## Description

### FIELD OF THE INVENTION

The invention relates generally to a device for skin treatment using radiation. More particularly, the invention relates to a device including a first solid state rare-earth laser exciting a second solid state laser for treatment of skin, such as hair removal, pigmented lesions, and tattoos. Surgical methods related to the treatment of vascular lesions, wrinkles, acne, skin tightening, and/or fat reduction do not form part of the invention.

### BACKGROUND OF THE INVENTION

Lasers are widely used in dermatological applications such as hair removal, removal of pigmented lesions, tattoos, vascular lesions, wrinkles, acne, and skin tightening. Dermatological laser treatments are typically based on selective targeting of a chromophore in the skin by an appropriate choice of wavelength and pulse duration of the laser light. Although lasers can provide better results than most other light sources, most medical laser devices use only a single wavelength of light. This limits the range of applications for which a particular medical laser can be used. Therefore, several different lasers can be needed to treat more than one skin condition.

In addition, solid-state lasers are typically pumped by flashlamps to get the large pulse energies required for creating the desired thermal profile in the skin. While such lasers can provide large output energies, the beam quality is generally poor and frequency conversion can be difficult.

US 5 757 839 discloses a Nd: YAG laser pumped by an alexandrite laser for industrial applications.

### SUMMARY OF THE INVENTION

Aspects and embodiments of the invention are set out according to the appended claims.

Rare earth solid state lasers can be used to excite another laser. Multi-laser systems based on laser pumping of one or more other lasers can provide two or more wavelengths with little additional cost, complexity or size. Also, a laser pumped laser generally can have better beam quality than a flashlamp pumped laser. Better beam quality can permit the generation of additional wavelengths by various methods of non-linear frequency conversion. Furthermore, laser pumping is particularly advantageous for leasers that are difficult to pump with other conventional sources like laser diodes and flashlamps.

The invention, in one embodiment, features a system or an apparatus for treatment of skin. A first solid state rare-earth laser can be used to excite a second solid state laser for treatment of skin disorders and conditions. Excitation of one laser by another enables generation of a new wavelength, along with an increase in brightness which further allows non-linear frequency conversion. The increase in brightness can also allow the beam to be focused to a small spot of high intensity laser energy that can be used to cut tissue in surgical applications.

In one aspect, the invention features a method for treating mammalian tissue including generating a first output beam of laser radiation using a first solid-state laser and directing a first part of the first output beam to a second solid-state laser. The method also includes generating a second output beam of laser radiation using the second solid-state laser based on excitation of a rare-earth doped gain medium, and directing the second output beam to a target region of mammalian tissue to treat a first condition of the mammalian tissue.

In another aspect, the invention features a laser system for treating skin including a first solid-state laser for producing a first output beam, a second solid state laser for producing a second output beam, and a delivery device. The second solid state is adapted to absorb a first part of the first output beam for generating excitation in a rare-earth doped gain medium to produce the second output beam. The delivery device directs the second output beam to a target region of skin, wherein the second output beam is for treating the skin.

In other examples, any of the aspects above or any apparatus or method described herein can include one or more of the following features. In various embodiments, the mammalian tissue can be skin. In one embodiment, treating the first condition can include removing black tattoos. In various embodiments, the laser system can further include beam shaping optics. The beam shaping optics can be used to direct the first part of the first output beam to the second solid-state laser, In one embodiment, the laser system can further include an optical fiber. The optical fiber can be used to direct the first part of the first output beam to the second solid-state laser. In some embodiments, the laser system further includes a handpiece. The handpiece can be used to direct the first part of the second output beam to the target region. In one embodiment, the method can further include directing a second part of the first output beam to the target region to treat a second condition. The second condition can include removing violet tattoos, blue tattoos, green tattoos, black tattoos, or any combination thereof.

In other examples, any of the aspects above or any apparatus or method described herein can include one or more of the following features. In various embodiments, the laser system can further include an output coupler mirror. The output coupler mirror can form a dual wavelength output beam from a second part of the first output beam that passes through the second solid-state laser and laser radiation from the second solid-state laser. In one embodiment, the method can further include directing the dual wavelength output beam to the target region to treat the first condition and a second condition. In various embodiments, the method can further can include generating, based on laser radiation received from the second solid-state laser, the second output beam using a nonlinear frequency converter. The laser system can include the nonlinear frequency converter. Treating the third condition can include removing red tattoos, orange tattoos, yellow tattoos, or any combination thereof. In some embodiments, the laser system can further include a q-switching element in the first solid-state laser to generate high peak power pulses of the first output beam.

In other examples, any of the aspects above or any apparatus or method described herein can include one or more of the following features. In various embodiments, the laser system can further include beam shaping optics adapted to receive laser radiation from the first solid-state laser for directing the first part of the first output beam to the second solid-state laser. The laser system can further include an optical fiber adapted to receive laser radiation from the first solid-state laser for directing the first part of the first output beam to the second solid-state laser. The delivery device can include a handpiece and the second solid state laser can be housed in the handpiece. The delivery device can include an output coupler mirror for forming a dual wavelength output beam from a second part of the first output beam that passes through the second solid-state laser and laser radiation from the second solid-state laser. In some embodiments, the laser system can further include a nonlinear frequency converter for generating, based on laser radiation received from the second solid-state laser, the second output beam. The laser system can further include a q-switching element in the first solid-state laser for generating high peak power pulses of the first output beam. The first solid-state laser can include a first host material. The first host material can include: sapphire, beryl, chrysoberyl, LiSAF, forsterite, or any combination thereof. The first host material can be doped with a transition metal, the transition metal comprising Cr or Ti. The second solid state laser can include a rare-earth doped gain medium. The rare earth doped gain medium can include: YANG, YAP, YVO4, YLF, YSGG, GSGG, FAP, GdVO4, KGd(WO₄)₂, SFAP, glass, ceramic, or any combination thereof. The rare-earth doped gain medium can be doped with rare-earth ions. Rare-earth ions can include: Nd, Yb, Er, Ho, Th, Sm, Ce, or any combination thereof.

In another aspect, the invention features a laser system for tattoo removal including a Q-switched alexandrine laser for producing a first output beam, a Nd:YAG laser for producing a second output beam, and a delivery device for directing the second output beam to a target region of skin. The wavelength of the first output beam is about 755 nm. The wavelength of the second output beam is about 1064 mn. The Nd:YAG laser is adapted to absorb the first output beam for generating excitation to produce the second output beam. The second output beam is for removing black tattoos.

In another aspect, the invention features a laser system for tattoo removal including a Q-switched alexandrine laser for producing a first output beam, a Nd:YAG laser for producing a second output beam, a KTP crystal for generating a third output beam, and a delivery device for directing the third output beam to a target region of skin. The wavelength of the first output beam is about 755 nm. The wavelength of the second output beam is about 1064 nm. The Nd:YAG laser is adapted to absorb the first output beam for generating excitation to produce the second output beam. The KTP crystal is adapted to absorb the second output beam for generating the third output beam. The wavelength of the third output beam is about 532 nm. The third output beam is for removing red tattoos, orange tattoos, yellow tattoos, or any combination of tattoos.

In other examples, any of the aspects above or any apparatus or method described herein can include one or more of the following features. In various embodiments, the first solid-state laser can include a transition-metal doped gain medium. In one embodiment, the first solid-state laser gain medium can be Alexandrite, ln various embodiments, the laser system can further include a nonlinear frequency converter for generating a third output beam from at least a part of the second output beam. The nonlinear frequency converter can be a second harmonic generation converter, a third harmonic generation converter, a fourth harmonic generation converter, an optical parametric oscillator, or a Raman shifting converter. In some embodiments, the first solid-state laser can include a first host material. The second solid-state laser can include a second host material. The second host material can include a crystalline structure. The crystalline structure can include: YAG, YAP, YVO4, YLF, YSGG, GSGG, FAP, GdVO₄, KG(I(WO₄)₂, or SFAP. The second host material can include an amorphous structure. The amorphous structure can include: glass or ceramic YAG. The second host material can be doped with a rare-earth ion selected from: Nd, Yb, Er, Ho or Th. The doping of the second host material can include a rare-earth ion selected from: Nd, Yb, Er, Ho or The. The doping of the second host crystal can include co-doping with one or more ions elected from: Cr, Nd, Yb, Er, Ho or Th. The rare-earth doped gain medium can be Nd:YAG.

In other examples, any of the aspects above or any apparatus or method described herein can include one or more of the following features. The second output beam can include a single pulse or a train of pulses, each pulse of duration between approximately 1 ns and approximately 500 ms. Each pulse can have an energy between about 1 microJoule and about 100 Joules. The first beam output can include a wavelength between about 400 nm and about 1000 nm. The second beam output can include a wavelength between about 400 nm and about 3000 nm.

In another aspect, the invention features a laser system for treating skin including a transition-metal laser producing a first output beam, and a rare-earth laser having a gain medium that absorbs at least a first part of the first output beam, thereby generating excitation in the gain medium and producing a second output beam.

In still another aspect, the invention features a laser system for treating skin including a flashlamp, a first gain medium excited by the flashlamp for producing a first output beam, a second gain medium excited by a part of the first output beam for producing a second output beam, a first coupling element for coupling the part of the first output beam to the second gain medium, and a second coupling element for coupling a part of the second output beam out of a cavity containing the second gain medium.

In still another aspect, the invention features a multi-wavelength laser system for treating skin including a flashlamp pumped Alexandrite laser producing a first output beam having a first wavelength and a first beam path, and an Alexandrite-pumped neodymium laser. The Alexandrite-pumped neodymium laser is movable from a first position not in the first beam path to a second position in said first beam path. The Alexandrite-pumped neodymium laser is also capable of absorbing at least some of the first output beam and producing a second output beam having a second wavelength and a second beam path coaxial with the first beam path. The neodymium laser includes a neodymium doped laser gain material.

In other examples, any of the aspects above or any apparatus or method described herein can include one or more of the following features. In various embodiments, the Alexandrine laser can include a KD*P q-switch, producing high peak power pulses. The laser system can further include a KTP second harmonic generator movable from a first position not in the second beam path to a position in the second beam path producing a third output beam having a third output wavelength and a third beam path coaxial with the second beam path. The neodymium laser can further include a Cr⁴⁺:YAG passive q-switch where the second output beam comprises a train of high peak power pulses. The laser system can further include a KTP second-harmonic generator movable from a first position not in the second beam path to a position in the second beam path producing a third output beam comprising a train of high peak power pulses having a third output wavelength and a third beam path coaxial with the second beam path.

In other examples, any of the aspects above or any apparatus or method described herein can include one or more of the following features, In various embodiments, the Alexandrite laser can include a KD*P q-switch, producing high peak power pulses. The handpiece can further include a KTP second-harmonic generator positioned in the second beam path, producing a third output beam having a third wavelength. The neodymium laser can further include a Cr⁴⁺:YAG passive q-switch producing a train of high peak power pulses, where the handpiece further comprises a KTP second-harmonic generator positioned in the second beam path, producing a third output beam having a third wavelength.

Advantages of the invention include one or more of the following. Techniques to generate new wavelengths from simple lamp pumped lasers will be very useful in this field by making devices in this field more versatile and cost effective. This invention also offers a method to improve beam quality of flashlamp pumped lasers without adding cost and complexity.

The details of one or more examples are set forth in the accompanying drawings and the description below. Further features, aspects, and advantages of the invention will become apparent from the description, the drawings, and the claims.

### BRIEF DESCRIPTION OF THE DRAWINGS

The advantages of the invention described above, together with further advantages, may be better understood by referring to the following description taken in conjunction with the accompanying drawings. The drawings are not necessarily to scale, emphasis instead generally being placed upon illustrating the principles of the invention.
FIG. 1 is a schematic drawing of a solid state laser pumped by a second solid state laser with q-switch and frequency converting elements.
FIG. 2 is a schematic drawing of a handpiece including a solid state laser pumped via an optical fiber by another solid state laser.

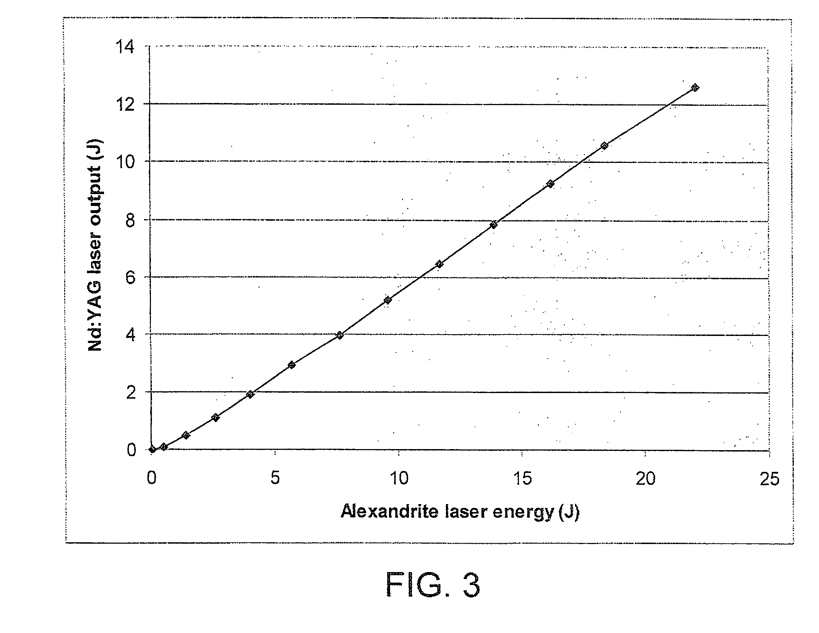

FIG. 3 is a graph of data from an experimental test of a rare-earth laser pumped by a transition metal laser.

### DESCRIPTION OF THE INVENTION

Lasers and other light sources are often used for the treatment of skin disorders and to produce cosmetic improvement in the appearance of the skin including the removal of hair from the skin. The heat produced by the light energy can modify structures within the skin and beneath the skin. Typical applications can include, for example, removal of hair, pigmented lesions, tattoos, vascular lesions, wrinkles, acne, skin tightening, and/or the like although surgical methods relating to the treatment of vascular lesions, wrinkles, acne and skin tightening do not form part of the invention. Applications can more generally also include treatment of mammalian tissue.

Dermatological laser treatments can be based on selectively targeting of a chromophore in or near the target structure by an appropriate choice of wavelength and pulse duration of the light. Lasers are often the preferred light source because a laser beam has a narrower wavelength bandwidth than light from other sources. A source with a narrow wavelength bandwidth can maximize the spectral selectivity of the target chromophore. In addition, lasers can be made with much shorter pulse durations than other light sources thereby maximizing the temporal selectivity of the targeted structure. The superior temporal selectivity makes lasers specially preferred for removing small targets like small vessels and tattoo pigment particles.

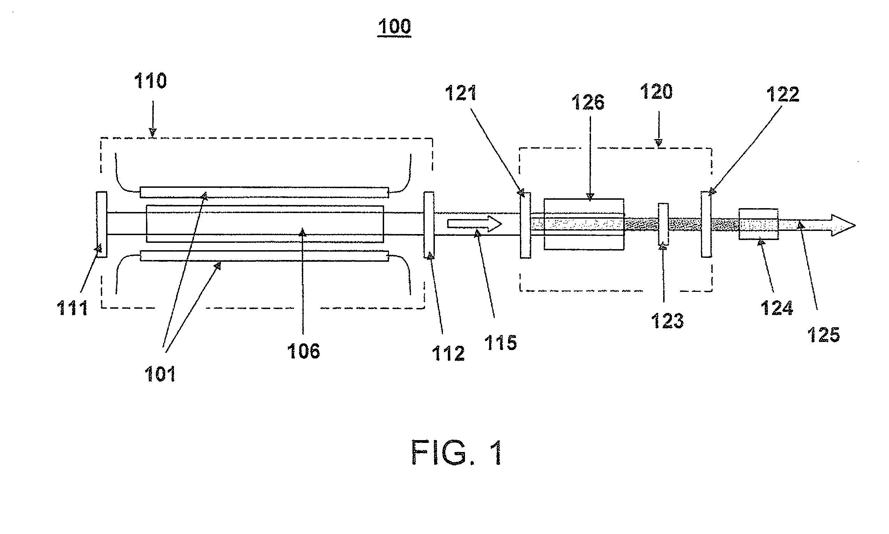

FIG. 1 is a schematic drawing of a laser system **100** including a solid state laser **110** pumping another solid state laser **120.** Laser gain materials **106** and **126** of the solid state lasers **110** and **120,** respectively, can be made in the shape of a round rod, but other configurations can also be used, such as, for example, slabs and cubes. The solid state laser **110** can be a transition metal laser such as, for example, an Alexandrine laser. The solid state laser **110** can be pumped by one or more flashlamps **101.** The cavity of the solid state laser 110 can include a high reflecting mirror **111** and an output coupler mirror **112.** The output coupler mirror **112** can couple an output beam **115** from the cavity of the first solid state laser **110.** The solid state laser **120** can be a rare-earth laser such as, for example, neodymium-YAG (Nd:YAG), The solid state laser **120** can be pumped by a portion of the output beam **115.** The output beam **115** can include either long, gain switched pulses, or short, Q-switched, pulses. The temporal profile of the output beam **125** from the laser cavity of the solid state laser 120 has been experimentally found to closely match the temporal profile of the output beam 115 for both long-pulse and/or short-pulse pumping. In addition, a Q-switched solid state laser **110** can be constructed so that long pulses can be produced by not energizing the Q-switching device.

The cavity of the solid state laser **120** can include a high reflecting mirror **121** and an output coupler mirror **122.** The output coupler mirror **122** can couple an output beam **125** from the cavity of the solid state laser **120.** In one embodiment, the cavity of the solid state laser **120** can include a q-switching element **123** such as, for example, a Cr⁴⁺:YAG (Cr:YAG) element. In another embodiment, a frequency doubling crystal such as, for example, KTP, can be positioned in the path of the output beam **125.**

In one embodiment, the solid state laser gain materials **106** and/or **126** can be directly coated on both ends with coatings of appropriate transmission and reflection properties to form the reflecting mirrors **111** and **121** and the output coupling mirrors **112** and 122. For a Alexandrite/Nd:YAG system, the reflecting mirror **121** and the output coupler mirror 122 can allow both double-pass pumping at about 755 nm and/or laser output at about 1064 nm. The absorption coefficient at about 755 nm can be about 2 cm⁻¹. Therefore, a solid state laser 120 with a gain medium 1.15 cm long can absorb approximately 99% of the pump energy in a double-pass configuration.

Coupling the output laser beam **115** of the solid state laser **110** into the gain material **126** of the solid state laser **120** can be accomplished in a variety of ways such as, for example, end-pumping, as illustrated in FIG. 1. In one embodiment, the laser gain material **126** of the solid state laser **120** can be made larger than the laser beam **115** so that the solid state laser **120** can be pumped directly without any manipulation of the pump beam **115.** The dimensions of the end of the solid state laser gain material **126** can be made slightly larger than the laser beam 115 so that the solid state laser **120** can be positioned directly in the path of the beam 115. Alternatively, the beam **115** can be shaped with mirrors, lenses, and/or other optical components to optimize the pump volume within the solid state laser **120.** In another embodiment, either solid state leaser **110** or solid state laser **120** can be mounted on a translation stage so that the system output beam **125** can be switched between about **755** nm and about 1064 nm simply by moving one of the solid state lasers. Translational stages can be used in systems where output beam 115 and/or output beam 125 are focused into an optical fiber for delivery of the treatment beam. Both output beams **115** and **125** can be more efficiently coupled into the fiber without any positional adjustment of the fiber coupling components (e.g., lens (not shown)).

Both Alexandrine and Nd:YAG lasers can be used in dermatological applications. Therefore, the selection of Alexandrite and Nd:YAG as the solid state lasers **110** and **120,** respectively, in the configuration illustrated in FIG. 1 can allow for complementary applications in a single system. The q-switched versions of both Alexandrite and Nd:YAG lasers, for example, can be used to remove tattoos. Due to the different optical absorption of the various colors of tattoo pigment, an Alexandrite laser can remove blue and green tattoos, while a Nd:YAG laser can remove black tattoos. The second harmonic of a Nd:YAG laser, about 532 nm, call remove red tattoos. Likewise, long-pulse versions of both Alexandrite and Nd:YAG lasers can be effective for hair removal. However, because the wavelengths of Alexandrite and Nd:YAG lasers have different optical absorption in melanin, an Alexandrite laser, at about 755 nm, can be better for treating light-skin patients while a Nd:YAG laser, at about 1064 non, can be better for dark-skin patients.

The absorption spectra of ND:YACT has a continuous band of lines ranging from about 725 nm to about 770 nm. Therefore, the non-tuned output of a free-running Alexandrite laser, at about 755 nm, can be used to pump a Nd:YAG laser. An important absorption line in the Nd:YAG is about 2 or 3 nm wide centered at about 755 nm. A stronger but narrower peak is centered at about 750 nm. Furthermore, there are wavelengths within the 725 nm to 770 nm band where excited state absorption can occur. However, there is very little excited state absorption at 755 nm, making it an attractive pump wavelength. The efficiency of the conversion of 755 nm to 1064 nm can be affected mostly by the quantum defect, which is about 30%. There can be another small loss, e.g., less than 5% percent in Nd:YAG, due to scattering effects.

Laser pumping can be particularly attractive for lasers that are difficult to pump with other conventional sources such as, for example, laser diodes and flashlamps. As an example, it can be difficult to generate high peak powers from Nd:YAG at 946 nm, which is one of the laser lines ofNd:YAG. The 946 nm is a three-level laser transition which requires a high pump rate to reach threshold. Flashlamp pumping can be inadequate due to poor brightness of the source, while diode lasers can essentially be continuous wave sources and not suitable for high peak power applications.

When end-pumped by a Q-switckted Alexandrite laser emitting a 50 ns pulse at 755 nm, for example, Nd:YAG can readily lase at 946 nm, emitting a similarly short pulse with hundreds of milli-Joules of energy, corresponding to several MW of peak power. In another example, when end-pumped with 25 Joule, 3 millisecond pulses from a free-running, gain-switched Alexandrite laser, an output of 6 Joules at 946 nm can be emitted by the Nd:YAG laser.

Although the pump beam can be absorbed by the gain medium, high absorption is not preferred in all embodiments. For example, some heat can be generated in a rare-earth gain medium by laser pumping, although the amount of heat can be much less than that deposited in the gain medium by flashlamp pumping. Nevertheless, the size of the gain medium can be chosen so that the heat can be removed fast enough to limit the temperature rise in the gain medium. The length of the gain medium and the magnitude of the absorption can be chosen so that the heat generation is distributed fairly evenly through the medium. In some cases, for example, the wavelength emitted by the pump laser can be tuned in order to adjust the absorption of the pump beam by the rare-earth laser.

Absorption spectra show that Nd:YVO₄, and Nd;GdVO₄ can also be excited by a free running Alexandrite laser. A tunable Alexandrite laser, from about 700 nm to about 818 nm, can be used to excite other laser gain materials such as, for example, Nd:YAP, Er:YAG, and/or Tm:YAG. Ti:sapphire, with a broader tunable range from about 700 nm to about 1050 nm, can also be used to pump Ho:YAG. The approximately 2.94 micron wavelength output of a Er:YAG laser has high optical absorption by water and can therefore be used to ablate a thin layer of the epidermis for removing some of the effects of aging and sun damage.

In some embodiments, Tm:YAG can provide laser output when pumped by a free-running Alexandrite laser. For example, when the thulium concentration is at 6 %, a gain length of two inches absorbed 95% of the pump energy with a double-pass pump configuration. The thulium laser can produce 8 Joules of approximately 2 micron laser output when pumped with a 25 Joule, 755 nm laser beam. In this case, about 15 Joules is deposited in the laser rod. The long length of the laser rod can provide sufficient surface area from which to extract the heat between pulses.

Like the Er:YAG above, the approximate 2 micron output of a Tm:YAG laser can be usable for improving the appearance of aged skin. Tm:YAG has the advantage that the wavelength of the output is tunable from about 1.93 microns to about 2.10 microns. The wavelength can be adjusted so that the depth of penetration in the skin can be selected over a range of about 110 micron to about 600 microns.

The laser system **100** can treat a patient at either or both of two wavelengths produced by solid state lasers **110** and **120** at the same or two different pulse duration. For example, a Q-switched Alexandrine laser without a tuning element as solid state laser **110** can produce approximately 50 nanosecond pulses at about 755 nm. The output beam **115** in this configuration can be used to treat the patient or to pump a solid state laser **120** such as Nd:YAG, in which case approximately 50 nanosecond pulses at about 1064 nm can be produced and can be used to treat the patient. In another embodiment, by not energizing or not including a Q-switching element in either cavity of solid state lasers **110** and **120,** the laser system **100** can also be used to treat the patient with long-pulses of either wavelength. In this configuration, the duration of the long pulses can be determined by the duration and output power of the energy pulses produced by the one or more flashlamps **101.**

The laser system **100** can realize one or more of the following advantages over a conventional Q-switched Nd:YAG laser. The duration of the pulse generated by a conventional Q-switched Nd:YAG laser is about 10 nanoseconds. At effective treatment energies, the peak power can be so high that it cannot be transmitted though an optical fiber without damaging the fiber. A conventional Q-switched Nd:YAG laser system, therefore, typically has expensive and inconvenient articulated-arm beam delivery systems to overcome this problem. The 50 nanosecond pulses generated, for example, by the laser system **100** can be transmitted by optical fiber, a simpler and less expensive design. Pulses generated by the laser system **100** can also be as long as 100 nanoseconds. Furthermore, a higher output energy is possible with laser system **100.** Q-switched operation can require that energy be stored in the laser cavity. But amplified spontaneous emission (ASE) can limit the amount of energy that can be stored in a Nd:YAG cavity, resulting in limited output. Gain switched operation in laser system **100,** however, does not have this problem because of the short duration of the pumped state of the laser gain material and of the high Q of the cavity.

In another embodiment, frequency doubling can be used to obtain about a 532 nm output beam **125.** For example, high peak power of 50 nanosecond Nd:YAG pulses from solid state laser **120** can enable efficient second-harmonic conversion of the about 1064 nm wavelength to about 532 nm. Generation of the second-harmonic can be accomplished with a frequency doubling crystal **124,** such as, for example, a KTP crystal. The output laser beam from solid state laser **120** laser can be polarized in order to maximize the efficiency of the wavelength conversion within the frequency doubling crystal **124.** A polarizing element can be installed in the cavity of solid state laser **120.** In an alternative embodiment, a different host material for solid state laser **120** can be selected. Both Nd:YVO₄, and Nd:GdYO₄ can produce linearly polarized outputs at about 1064 nm and about 1063 nm, respectively. The long-pulse 1064 nm beam may not be efficiently frequency doubled because the peak power is low. This problem can be overcome by repetitively Q-switching the solid state laser 120 or the solid-state laser **1**10. Either active or passive Q-switching can accomplish repetitive Q-switching. A Cr⁴⁺:YAG passive Q-switch can also be placed in the resonator cavity of solid state laser 120 to generate a train of high peak power pulses that can be efficiently frequency doubled to about 532 nm.

Another method of coupling the pump beam 115 into the solid state laser 120 can include using a fiber optic coupling system. First, one or more lenses or other optical components can converge at least a portion of the pump beam 115 into an optical fiber (not shown) though which a portion of the pump beam **115** can be transmitted. The beam exiting the distal end of the optical fiber can be a divergent beam, which can be directed into the gain material of solid state laser 120 or it can be shaped and/or collimated by one or more lenses or other optical components before being directed into the gain material of solid state laser 120.

In one embodiment, a diode laser output at 808 nm can be used for hair removal and for pumping a ND:YAG laser. The diode laser system can include an optical system to optimize the divergence of the diode laser beam for treating hair and pumping the Nd:YAG laser.

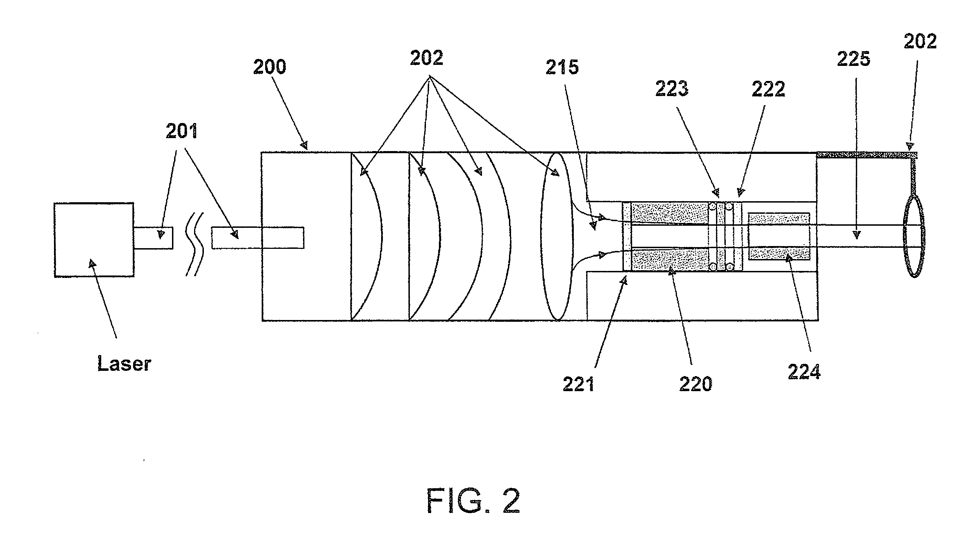

FIG. 2 is a schematic drawing of a handpiece 200 including a solid state laser 220 pumped via all optical fiber 201 by another solid state laser. In various embodiments, the handpiece 200 can include one or more optical components 202 for coupling at least a portion of the output beam 215 from the optical fiber 201 into the cavity of the solid state laser 220. The cavity of the solid state laser 220 can include a high reflecting mirror 221 and an output coupler mirror 222. In one embodiment, the cavity of the solid state laser 220 can include a Q-switching element 223. In another embodiment, the handpiece 200 can include a frequency doubling crystal 234. The output beam 225 of the handpiece can further be optical modified by an optical component 202 before it is used to treat the skin of a patient. In yet a further embodiment, the solid state laser 220 in the handpiece 200 can include an Er:YAG laser. In another embodiment, the solid state laser 220 in the handpiece 200 can include a Tm:YAG laser with or without a wavelength tuning device such as, for example, a birefringent tuner.

An alexandrite-pumped-neodymium laser system can be useful for a variety of medical applications, and in particular, dermatology. The three treatment wavelengths and two pulse durations capable of being produced by the laser system 100 can provide a range of six spectrally and temporally selective treatment modes thereby making this system clinically effective for a large range of medical conditions. The efficient conversion of electrical input energy to laser output energy at all three wavelengths can allow the design of competitively sized and priced laser products. Products based on sub-sets of the elements described herein can also be clinically useful and commercially viable.

To minimize thermal injury to tissue surrounding an eye and/or to an exposed surface of the target region, the delivery system (e.g., handpiece 200) can include a cooling system for cooling before, during and/or after delivery of radiation, Cooling can include contact conduction cooling, evaporative spray cooling, convective air flow cooling, or a combination of the aforementioned, In one embodiment, the handpiece 200 includes a skin contacting portion that can be brought into contact with the skin. The skin contacting portion can include a sapphire or glass window and a fluid passage containing a cooling fluid. The cooling fluid can be a fluorocarbon type cooling fluid, which can be transparent to the radiation used. The cooling fluid can circulate through the fluid passage and past the window to cool the skin.

A spray cooling device can use cryogen, water, or air as a coolant. In one embodiment, a dynamic cooling device can be used to cool the skin (e.g., a DCD available from Candela Corporation). For example, the delivery system can include tubing for delivering a cooling fluid to the handpiece 200. The tubing can be connected to a container of a low boiling point fluid, and the handpiece 200 can include a valve for delivering a spurt of the fluid to the skin. Heat can be extracted from the skin by virtue of evaporative cooling of the low boiling point fluid. The fluid can be a non-toxic substance with high vapor pressure at normal body temperature, such as a Freon or tetrafluoroethane.

The invention has been described in terms of particular embodiments. The alternatives described herein are examples for illustration only and not to limit the alternatives in any way. The steps of the invention can be performed in a different order and still achieve desirable results. Other embodiments are within the scope of the following claims.

## Claims

1. A method of treating mammalian tissue, comprising the following steps:
generating a first output beam (115) of laser radiation of about 755 nm using an alexandrite laser (110);
directing the first output beam (115) to a delivery device for directing a system output beam of laser radiation to a target region of mammalian tissue for non-therapeutic hair removal or tattoo removal;
positioning a second solid-state laser (120) mounted on a translational stage into the path of the first output beam (115), the second solid-state laser being a neodymium laser producing a second output beam (125) of laser radiation of about 1064 nm based on excitation from the absorbed first output beam (115) in a rare-earth doped gain medium; and
directing the second output beam (125) to the delivery device for directing the target output beam to the target region of mammalian tissue for hair removal or tattoo removal, wherein long-pulse alexandrite and neodymium lasers are used for hair removal, and q-switched versions of these lasers are used for tattoo removal.

2. The method of claim 1, wherein the mammalian tissue is skin.

3. The method of claim 1, , wherein the second output is used for removing black tattoos.

4. The method of claim 1, wherein a part of the first output beam (115) is used for removing violet tattoos, blue tattoos, green tattoos, black tattoos, or any combination thereof.

5. The method of claim 1, further comprising the step of using beam shaping optics for directing a part of the first output beam (115) to the second solid-state laser (120).

6. The method of claim 1, wherein the delivery device comprises an optical fiber used to direct a part of the first output beam to the second solid state laser (120).

7. The method of claim 1, wherein the delivery device comprises a handpiece (200) for directing the second output beam (125) to the target region.

8. The method of claim 1, further comprising the steps of
forming, using an output coupler mirror (122), a dual wavelength output beam from a part of the first output beam (115) that passes through the second solid-state laser (120) and laser radiation from the second solid-state laser (120); and
directing the dual wavelength output beam to the target region to remove a first condition and a second condition.

9. The method of claim 1, further comprising the step of arranging a nonlinear frequency converter to generate a target output beam from at least a part of the second output beam for removing red tattoos, orange tattoos, yellow tattoos, or any combination thereof.

10. The method of claim 1 wherein the first solid-state laser uses a q-switching element to generate high peak power pulses of the first output beam.

11. A multiple laser system for treating mammalian tissue, comprising:
a first solid state laser (110) being an alexandrite laser for producing a first output beam (115) of laser radiation of about 755 nm;
a second solid-state laser being a neodymium (120) pumped by the alexandrite laser for producing a second output beam (125) of laser radiation of about 1064 nm;
a delivery device for directing a system output beam of laser radiation to a target region of mammalian tissue, the system output beam being the first output beam (115) or the second output beam (125); and
a translation stage on which the first solid-sate laser (110) or the second solid-state laser (120) is mounted, wherein the system is configured to switch the system output beam between the first output beam (115) and the second output beam (125) due to moving one of the solid-state lasers.

12. The system of claim 11, further comprising beam shaping optics adapted to receive laser radiation from the first solid state laser for directing the first part of the first output beam (115) to the second solid-state laser (120).

13. The system of claim 11, the delivery device comprising an output coupler mirror (122) for forming a dual wavelength output beam from a second part of the first output beam (115) that passes through the second solid-state laser (120) and laser radiation from the second solid-state laser (120).

14. The system of claim 11, further comprising a nonlinear frequency converter arranged to generate a third output beam from at least a part of the second output beam (125).

15. The system of claim 11, the first solid-state laser (110) comprising a q-switching element for generating high peak power pulses of the first output beam (115).

## Patentansprüche

1. Ein Verfahren zur Behandlung von Säugetiergewebe, das die folgenden Schritte umfasst:
Erzeugen eines ersten Ausgangsstrahls (115) einer Laserstrahlung von ca. 755 nm unter Verwendung eines Alexandrit-Lasers (110);
Lenken des ersten Ausgangsstrahls (115) zu einer Abgabevorrichtung, um einen Systemausgangsstrahl einer Laserstrahlung auf eine Zielregion von Säugetiergewebe zur nichttherapeutischen Haar- oder Tätowierungsentfernung zur richten;
Positionieren eines zweiten Festkörperlasers (120), der auf einer Verschiebeplattform montiert ist, in den Pfad des ersten Ausgangsstrahls (115), wobei der zweite Festkörperlaser ein Neodym-Laser ist, der einen zweiten Ausgangsstrahl (125) einer Laserstrahlung von ca. 1064 nm erzeugt, basierend auf der Anregung aus dem absorbierten ersten Ausgangsstrahl (115) in einem Seltenerd-dotierten Verstärkungsmedium; und
Lenken des zweiten Ausgangsstrahls (125) zur Abgabevorrichtung, um den Zielausgangsstrahl auf die Zielregion des Säugetiergewebes zur Haar- oder Tätowierungsentfernung zu richten, wobei langgepulste Alexandrit- und Neodym-Laser zur Haarentfernung und gütegeschaltete Laser zur Entfernung von Tätowierungen eingesetzt werden.

2. Das Verfahren von Anspruch 1, wobei das Säugetiergewebe Haut ist.

3. Das Verfahren von Anspruch 1, wobei des zweiten Ausgangsstrahls verwendet wird, um schwarze Tätowierungen zu entfernen.

4. Das Verfahren von Anspruch 1, wobei ein Teil des ersten Ausgangsstrahls (115) herangezogen wird, um violette Tätowierungen, blaue Tätowierungen, grüne Tätowierungen, schwarze Tätowierungen oder eine Kombination davon zu entfernen.

5. Das Verfahren von Anspruch 1, das überdies den Schritt der Verwendung von Strahlformungsoptiken umfasst, um einen Teil des ersten Ausgangsstrahls (115) zum zweiten Festkörperlaser (120) zu lenken.

6. Das Verfahren von Anspruch 1, wobei die Abgabevorrichtung eine optische Faser umfasst, die verwendet wird, um einen Teil des ersten Ausgangsstrahls zum zweiten Festkörperlaser (120) zu lenken.

7. Das Verfahren von Anspruch 1, worin die Abgabevorrichtung ein Handstück (200) umfasst, um den zweiten Ausgangsstrahl (125) auf die Zielregion zur richten.

8. Das Verfahren von Anspruch 1, das überdies die folgenden Schritte umfasst:
Bilden eines Dual-Wellenlängen-Ausgangsstrahls unter Verwendung eines Auskoppelspiegels aus einem Teil des ersten Ausgangsstrahls (115), der durch den zweiten Festkörperlaser (120 und Laserstrahlung vom zweiten Festkörperlaser (120) geht; und
Richten des Dual-Wellenlängenstrahls auf die Zielregion, um einen ersten Zustand und einen zweiten Zustand zu entfernen.

9. Das Verfahren von Anspruch 1, überdies umfassend den Schritt des Anordnens eines nichtlinearen Frequenzkonverters, um einen Zielausgangsstrahl aus zumindest einem Teil des zweiten Ausgangsstrahls zum Entfernen von roten Tätowierungen, orangen Tätowierungen, gelben Tätowierungen oder Kombinationen davon zu erzeugen.

10. Das Verfahren von Anspruch 1, wobei der erste Festkörperlaser ein gütegeschaltetes Element verwendet, um Impulse des ersten Ausgangsstrahls mit hohen Leistungsspitzen zu erzeugen.

11. Ein Mehrfachlasersystem zur Behandlung von Säugetiergewebe, umfassend:
einen ersten Festkörperlaser (110), der ein Alexandrit-Laser ist, um einen ersten Ausgangsstrahl (115) einer Laserstrahlung von ca. 755 nm zu erzeugen;
einen zweiten Festkörperlaser, der ein Neodym-Laser (120) ist, welcher vom Alexandrit-Laser gepumpt wird, um einen zweiten Ausgangsstrahl (125) einer Laserstrahlung von ca. 1064 nm zu erzeugen;
eine Abgabevorrichtung, um einen Systemausgangsstrahls einer Laserstrahlung auf eine Zielregion von Säugetiergewebe zur richten, wobei der Systemausgangsstrahl der erste Ausgangsstrahl (115) oder der zweite Ausgangsstrahl (125) ist; und
eine Verschiebeplattform, auf der der erste Festkörperlaser (110) und der zweite Festkörperlaser (120) montiert sind, wobei das System konfiguriert ist, um den Systemausgangsstrahl durch das Bewegen von einem der Festkörperlaser zwischen dem ersten Ausgangsstrahl (115) und dem zweiten Ausgangsstrahl (125) zu schalten.

12. Das System von Anspruch 11, das überdies Strahlformungsoptiken umfasst, die geeignet sind, um eine Laserstrahlung vom ersten Festkörperlaser zu empfangen, sodass der erste Teil des ersten Ausgangsstrahls (115) zum zweiten Festkörperlaster (120) gelenkt wird.

13. Das System von Anspruch 11, wobei die Abgabevorrichtung einen Auskoppelspiegel (122) umfasst, um einen Dual-Wellenlängen-Ausgangsstrahls aus einem zweiten Teil des ersten Ausgangsstrahls (115) zu bilden, der durch den zweiten Festkörperlaser (120) und Laserstrahlung vom zweiten Festkörperlaser (120) geht.

14. Das System von Anspruch 11, das überdies einen nichtlinearen Frequenzkonverter umfasst, der angeordnet ist, um einen dritten Ausgangsstrahl von mindestens einem Teil des zweiten Ausgangsstrahls (125) zu erzeugen.

15. Das System von Anspruch 11, wobei der erste Festkörperlaser (110) ein gütegeschaltetes Element umfasst, um Impulse des ersten Ausgangsstrahls (115) mit hohen Leistungsspitzen zu erzeugen.

## Revendications

1. Un procédé de traitement d'un tissu de mammifère, comprenant les opérations suivantes :
la génération d'un premier faisceau de sortie (115) de rayonnement laser d'environ 755 nm au moyen d'un laser à alexandrite (110),
la direction du premier faisceau de sortie (115) vers un dispositif d'administration destiné à la direction d'un faisceau de sortie système de rayonnement laser vers une zone cible d'un tissu de mammifère pour une élimination non thérapeutique de tatouages ou de poils,
le positionnement d'un deuxième laser à solide (120) monté sur un étage de translation dans le trajet du premier faisceau de sortie (115), le deuxième laser à solide étant un laser au néodyme produisant un deuxième faisceau de sortie (125) de rayonnement laser d'environ 1064 nm basé sur une excitation provenant du premier faisceau de sortie absorbé (115) dans un milieu de gain dopé aux terres rares, et
la direction du deuxième faisceau de sortie (125) vers le dispositif d'administration destiné à la direction du faisceau de sortie cible vers la zone cible d'un tissu de mammifère pour une élimination de poils ou de tatouages, où des lasers au néodyme ou à alexandrite à impulsions longues sont utilisés pour l'élimination de poils et des versions à commutation q de ces lasers sont utilisées pour l'élimination de tatouages.

2. Le procédé selon la Revendication 1, où le tissu de mammifère est de la peau.

3. Le procédé selon la Revendication 1, où la deuxième faisceau de sortie est utilisée pour l'élimination de tatouages noirs.

4. Le procédé selon la Revendication 1, où une partie du premier faisceau de sortie (115) est utilisée pour l'élimination de tatouages violets, de tatouages bleus, de tatouages verts, de tatouages noirs, ou de toute combinaison de ceux-ci.

5. Le procédé selon la Revendication 1, comprenant en outre l'opération d'utilisation d'optiques de mise en forme de faisceau de façon à diriger une partie du premier faisceau de sortie (115) vers le deuxième laser à solide (120).

6. Le procédé selon la Revendication 1, où le dispositif d'administration comprend une fibre optique utilisée pour diriger une partie du premier faisceau de sortie vers le deuxième laser à solide (120).

7. Le procédé selon la Revendication 1, où le dispositif d'administration comprend un embout à main (200) destiné à la direction du deuxième faisceau de sortie (125) vers la zone cible.

8. Le procédé selon la Revendication 1, comprenant en outre les opérations suivantes :
la formation, au moyen d'un miroir de coupleur de sortie (122), d'un faisceau de sortie à double longueur d'onde à partir d'une partie du premier faisceau de sortie (115) qui passe au travers du deuxième laser à solide (120) et d'un rayonnement laser provenant du deuxième laser à solide (120), et
la direction du faisceau de sortie à double longueur d'onde vers la zone cible de façon à éliminer un premier état et un deuxième état.

9. Le procédé selon la Revendication 1, comprenant en outre l'opération d'agencement d'un convertisseur de fréquence non linéaire de façon à générer un faisceau de sortie cible à partir d'au moins une partie du deuxième faisceau de sortie pour l'élimination de tatouages rouges, tatouages oranges, tatouages jaunes ou toute combinaison de ceux-ci.

10. Le procédé selon la Revendication 1 où le premier laser à solide utilise un élément à commutation q de façon à générer des impulsions à puissance de crête élevée du premier faisceau de sortie.

11. Un système à lasers multiples pour le traitement d'un tissu de mammifère comprenant :
un premier laser à solide (110) qui est un laser à alexandrite pour la production d'un premier faisceau de sortie (115) de rayonnement laser d'environ 755 nm,
un deuxième laser à solide qui est un laser à néodyme (120) pompé par le laser à alexandrite pour la production d'un deuxième faisceau de sortie (125) de rayonnement laser d'environ 1064 nm,
un dispositif d'administration destiné à la direction d'un faisceau de sortie système de rayonnement laser vers une zone cible d'un tissu de mammifère, le faisceau de sortie système étant le premier faisceau de sortie (115) ou le deuxième faisceau de sortie (125), et
un étage de translation sur lequel le premier laser à solide (110) ou le deuxième laser à solide (120) est monté, où le système est configuré de façon à commuter le faisceau de sortie système entre le premier faisceau de sortie (115) et le deuxième faisceau de sortie (125) du fait du déplacement de l'un des lasers à solide.

12. Le système selon la Revendication 11, comprenant en outre des optiques de mise en forme de faisceau adaptés de façon à recevoir un rayonnement laser provenant du premier laser à solide et destiné à la direction de la première partie du premier faisceau de sortie (115) vers le deuxième laser à solide (120).

13. Le système selon la Revendication 11, le dispositif d'administration comprenant un miroir de coupleur de sortie (122) destiné à la formation d'un faisceau de sortie à double longueur d'onde à partir d'une deuxième partie du premier faisceau de sortie (115) qui passe au travers du deuxième laser à solide (120) et du rayonnement laser provenant du deuxième laser à solide (120).

14. Le système selon la Revendication 11, comprenant en outre un convertisseur de fréquence non linéaire agencé de façon à générer un troisième faisceau de sortie provenant d'au moins une partie du deuxième faisceau de sortie (125).

15. Le système selon la Revendication 11, le premier laser à solide (110) comprenant un élément à commutation q pour la génération d'impulsions à puissance de crête élevée du premier faisceau de sortie (115).
